(19) Europäisches Patentamt
European Patent Office
Office européen des brevets

(11) EP 0 969 832 B1

(12) **FASCICULE DE BREVET EUROPEEN**

(45) Date de publication et mention de la délivrance du brevet:
**04.07.2001 Bulletin 2001/27**

(21) Numéro de dépôt: **98917259.8**

(22) Date de dépôt: **27.03.1998**

(51) Int Cl.[7]: **A61K 31/415**, A61K 9/16, A61K 9/20

(86) Numéro de dépôt international:
**PCT/FR98/00631**

(87) Numéro de publication internationale:
**WO 98/43636 (08.10.1998 Gazette 1998/40)**

(54) **COMPOSITION PHARMACEUTIQUE POUR ADMINISTRATION ORALE D'UN DERIVE DU N-PIPERIDINO-3 -PYRAZOLECARBOXAMIDE, DE SES SELS ET DE LEURS SOLVATES**

PHARMAZEUTISCHE ZUSAMMENSETZUNG ZUR ORALEN VERABREICHUNG VON EINEM N-PIPERIDIN-3-PYRAZOLCARBOXAMID-DERIVAT, DESSEN SALZEN UND DEREN SOLVATEN

PHARMACEUTICAL COMPOSITION FOR ORAL ADMINISTRATION OF A N-PIPERIDINO-3-PYRAZOLECARBOXAMIDE DERIVATIVE, ITS SALTS AND THEIR SOLVATES

(84) Etats contractants désignés:
**AT BE CH DE DK ES FI FR GB GR IE IT LI LU MC NL PT SE**
Etats d'extension désignés:
**LT LV SI**

(30) Priorité: **28.03.1997 FR 9703835**

(43) Date de publication de la demande:
**12.01.2000 Bulletin 2000/02**

(73) Titulaire: **SANOFI-SYNTHELABO**
**75013 Paris (FR)**

(72) Inventeurs:
- **ABRAMOVICI, Bernard**
  **F-34990 Juvignac (FR)**
- **CONDAMINE, Christian**
  **F-34690 Fabrègues (FR)**
- **GROMENIL, Jean-Claude**
  **F-34560 Montbazin (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 656 354**
**WO-A-96/22080**
**US-A- 5 087 454**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 969 832 B1

Printed by Jouve, 75001 PARIS (FR)

## Description

La présente invention a pour objet une composition pharmaceutique pour l'administration orale du N-pipéridino-5-(4-chlorophényl)-1-(2,4-dichlorophényl)-4-méthylpyrazole-3-carboxamide de formule :

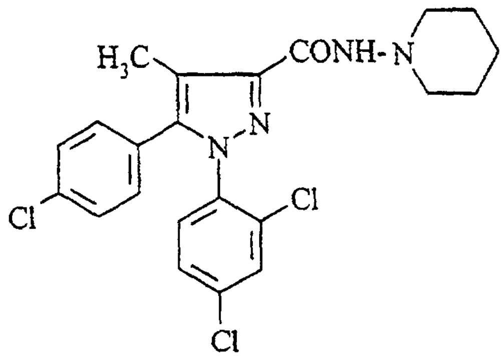

de ses sels pharmaceutiquement acceptables et de leurs solvates, ci-après dénommés composés de formule (I).

Les composés de formule (I) et leur mode de préparation sont décrits dans la demande de brevet européenne EP 656 354.

Le N-pipéridino-5-(4-chlorophényl)-1-(2,4-dichlorophényl)-4-méthylpyrazole-3-carboxamide également connu par le nom de code SR 141716 et appelé composé A dans la description ci-après, est tout particulièrement préféré pour la composition pharmaceutique selon la présente invention.

Les propriétés pharmacologiques des composés de formule (I), qui sont des antagonistes sélectifs des récepteurs aux cannabinoïdes centraux CBI, ont été relatées, notamment dans la publication de M. Rinaldi-Carmona et al., FEBS Letters, 1994, 240-244.

Pour administrer de tels composés par voie orale, il est nécessaire qu'ils présentent une bonne absorption, ce qui implique à la fois une bonne solubilité en milieu aqueux et une bonne capacité à traverser la membrane intestinale (M. Rowland et T.N. Tozer in Clinical Pharmacokinetics, concepts and applications, Lea and Fehiger ed., 1989, 2nd edition, p. 113-130).

Pour évaluer la perméabilité épithéliale des composés, on utilise la lignée cellulaire Caco-2 qui a la particularité de se différencier in-vitro pour former une monocouche épithéliale (Crit. Rev. Ther. Drug Carrier System, 1991, 8 (4), 105-330). Sur ce modèle, la perméabilité du composé A mis en solution dans le diméthylsulfoxyde (DMSO) est élevée, ce qui montre sa bonne aptitude à être absorbé au niveau intestinal, lorsqu'il est en solution.

Par ailleurs, le caractère hydrophobe des composés de formule (I) est très fortement marqué. Ainsi, on a observé que le composé A n'est pas mouillable dans l'eau et que ce composé et ses sels sont pratiquement insolubles dans l'eau quel que soit le pH. Ces composés sont solubles dans les alcools et dans les glycols, plus particulièrement dans les polyéthylèneglycols (PEG).

Cependant, lorsque les solutions obtenues avec un alcool ou un glycol sont diluées en milieu aqueux, le composé de formule (I) précipite, en raison de son fort caractère hydrophobe.

Les composés de formule (I) et en particulier le composé A sont peu électrostatiques. La micronisation peut s'opérer avec un bon rendement (environ 85 %) et permet d'obtenir des particules d'environ 1 micron. Les contrôles analytiques effectués après micronisation montrent qu'il n'y a pas de modification de la forme cristalline.

En étudiant la mouillabilité, on a constaté que la vitesse de pénétration de l'eau dans un lit de poudre formulé par granulation humide est beaucoup plus élevée que celle mesurée dans un lit de poudre obtenu par mélange à sec. L'étude des effets de l'incorporation d'agents mouillants a montré qu'un alkylsulfate de sodium à faible concentration augmente nettement la mouillabilité.

Par ailleurs, on a trouvé que la présence d'un agent de désagrégation, comme la carboxyméthylcellulose sodique réticulée, dans la formulation permet d'améliorer la cinétique de dissolution.

De façon surprenante, on a trouvé qu'en associant dans la même formulation un alkylsulfate de sodium et un agent de désagrégation, on obtient rapidement une dissolution complète de la formulation, et ce, avec une bonne reproductibilité des résultats.

Ainsi, selon un de ses aspects, la présente invention a pour objet une composition pharmaceutique pour l'administration orale d'un composé de formule (I) comprenant

- 0,5 % à 20 % en poids d'un composé de formule (I) sous forme micronisée,
- 0,05 % à 0,5 % en poids d'un alkylsulfate de sodium,

- 2,5 % à 10 % en poids d'agent de désagrégation,

et des excipients pharmaceutiques, ladite composition étant formulée par granulation humide.

Par granulation humide on entend l'opération pharmaceutique qui permet, à l'aide d'un liquide de granulation, de densifier un mélange de poudres contenant le principe actif. ledit mélange constituant la phase interne de la formulation, la masse humide ainsi obtenue étant séchée puis calibrée avant l'addition des ingrédients constituant la phase externe de ladite formulation.

Selon la présente invention, par alkylsulfate de sodium on entend un ($C_8$-$C_{12}$)alkylsulfate de sodium comme par exemple l'octylsulfate de sodium ou préférentiellement le laurylsulfate de sodium.

Selon la présente invention, par agent de désagrégation, on entend la cellulose ou les dérivés cellulosiques, tels que la carboxyméthylcellulose sodique, la carboxyméthylcellulose sodique réticulée, la crospovidone, l'amidon prégélatinisé, le carboxyméthylamidon sodique ; la carboxyméthylcellulose sodique réticulée étant un agent de désagrégation préféré.

Les compositions pharmaceutiques selon la présente invention peuvent se présenter sous forme de gélules, de comprimés, de sachets ou de poudres, préférentiellement sous forme de gélules.

Les excipients pharmaceutiques utiles pour la composition pharmaceutique selon la présente invention comprennent notamment un diluant, un liant et un lubrifiant. On peut également ajouter un agent d'écoulement, un antiadhérent et éventuellement un colorant et/ou un aromatisant.

Le diluant utilisé dans la composition de la présente invention peut être un ou plusieurs composés qui sont capables de densifier le principe actif pour obtenir la masse désirée. Les diluants préférés sont les phosphates minéraux, tels que les phosphates de calcium ; les sucres, tels le lactose hydraté ou anhydre, le mannitol ; et la cellulose ou les dérivés cellulosiques, comme par exemple la cellulose microcristalline, l'amidon, l'amidon de maïs ou l'amidon prégélatinisé. Tout particulièrement, on préfère le lactose monohydrate, le mannitol, la cellulose microcristalline et l'amidon de maïs utilisés seuls ou en mélange tels que par exemple un mélange de lactose monohydrate et d'amidon de maïs.

Le liant employé dans la composition de la présente invention peut être un ou plusieurs composés qui sont capables de densifier un composé de formule (I) en le transformant en particules plus grosses et plus denses, ayant un meilleur écoulement. Les liants préférés sont l'acide alginique, l'alginate de sodium ; la cellulose et les dérivés cellulosiques, tels que la carboxyméthylcellulose sodique, l'éthylcellulose, l'hydroxyéthylcellulose, l'hydroxypropylcellulose, l'hydroxypropylméthylcellulose ou la méthylcellulose ; la gélatine ; les polymères d'acide acrylique ; la povidone, par exemple la povidone K-30 ; la povidone K-30 étant un liant tout particulièrement préféré. Le liant est présent dans une proportion pondérale de 1 % à 10 % dans la composition pharmaceutique selon l'invention.

Le lubrifiant employé dans la composition de la présente invention peut être un ou plusieurs composés qui sont capables d'empêcher les problèmes liés à la préparation de formes sèches, comme les problèmes de collage et/ou de grippage qui surviennent au niveau des machines lors de la compression ou du remplissage. Les lubrifiants préférés sont des acides gras ou des dérivés d'acides gras comme le stéarate de calcium, le monostéarate de glycéryle, le palmitostéarate de glycéryle, le stéarate de magnésium, le laurylsulfate de sodium, le stéarylfumarate de sodium, le stéarate de zinc, ou l'acide stéarique ; les huiles végétales hydrogénées, par exemple l'huile de ricin hydrogénée ; les polyalkylèneglycols, notamment le polyéthylèneglycol ; le benzoate de sodium ou le talc. Selon la présente invention, le stéarate de magnésium est préféré. Le lubrifiant est présent dans une proportion pondérale de 0,2 % à 5 % dans la composition pharmaceutique selon l'invention.

L'antiadhérent éventuellement employé dans la composition de la présente invention peut être un ou plusieurs composés qui sont capables de réduire le caractère collant de la formulation, par exemple d'empêcher l'adhérence aux surfaces métalliques. Les antiadhérents préférés sont des composés qui contiennent du silicium, par exemple de la silice ou du talc. L'antiadhérent peut être présent dans une proportion pondérale de 0 à 5 % dans la composition pharmaceutique selon l'invention.

L'agent d'écoulement éventuellement employé dans la composition de la présente invention peut être un ou plusieurs composés qui sont capables de faciliter l'écoulement de la formulation préparée. Les agents d'écoulement préférés sont des composés qui contiennent du silicium, par exemple de la silice colloïdale anhydre ou de la silice précipitée. L'agent d'écoulement peut être présent dans une proportion pondérale de 0 à 15 % dans la composition pharmaceutique selon l'invention.

Selon la présente invention, les compositions pharmaceutiques sont préparées par un procédé de granulation humide. Ainsi pour la phase interne, on mélange à température ambiante le principe actif, le diluant, le liant, l'agent de désagrégation, l'alkylsulfate de sodium, et éventuellement le colorant puis on mouille avec le liquide de granulation. La masse humide obtenue est séchée, puis calibrée. Aux grains secs calibrés, on ajoute alors le ou les ingrédients de phase externe à savoir le lubrifiant, et éventuellement l'antiadhérent, l'agent d'écoulement et le cas échéant le colorant et/ou l'aromatisant.

L'eau purifiée est utilisée comme liquide de granulation.

Selon un mode de réalisation préférentiel l'alkylsulfate de sodium est ajouté à l'eau purifiée pour procéder à la granulation humide.

De façon particulière, la présente invention concerne une composition pharmaceutique pour l'administration orale comprenant :

- 0,5 % à 20 % en poids du composé A sous forme micronisée,
- 0,05 % à 0,5 % en poids de laurylsulfate de sodium,
- 2,5 % à 10 % en poids de carboxyméthylcellulose sodique réticulée,

et des excipients pharmaceutiques, ladite composition étant formulée par granulation humide.

De façon préférentielle, la présente invention a pour objet une composition pharmaceutique pour l'administration orale, formulée par granulation humide et contenant :

- 0,5 % à 20 % en poids du composé A sous forme micronisée,
- 0,1 % en poids de laurylsulfate de sodium,
- 5 % en poids de carboxyméthylcellulose sodique réticulée,
- 1 % à 10 % en poids de liant,
- 0,2 % à 5 % en poids de lubrifiant,

et un diluant en quantité suffisante pour 100 %.

Tout particulièrement, la présente invention est relative à des compositions pharmaceutiques sous forme de gélules, préparées par granulation humide, ayant l'une des formulations suivantes exprimées en pourcentage pondéral :

| | |
|---|---|
| i) Phase interne | |
| composé A micronisé<br>amidon de maïs<br>lactose monohydrate 200 mesh<br>povidone K 30<br>carboxyméthylcellulose sodique réticulée | 0,59 %<br>30 %<br>60,78 %<br>2,53 %<br>5 % |
| Granulation | |
| laurylsulfate de sodium<br>eau purifiée | 0,1 %<br>Q.S. |
| Phase externe | |
| stéarate de magnésium | 1% |
| ii) Phase interne | |
| composé A micronisé<br>amidon de maïs<br>lactose monohydrate 200 mesh<br>povidone K 30<br>carboxyméthylcellulose sodique réticulée | 5,88 %<br>30 %<br>55,49 %<br>2,53 %<br>5 % |
| Granulation | |
| laurylsulfate de sodium<br>eau purifiée | 0,1%<br>Q.S. |
| Phase externe | |
| stéarate de magnésium | 1 % |
| iii) Phase interne | |
| composé A micronisé<br>amidon de maïs<br>lactose monohydrate 200 mesh | 17,64 %<br>30 %<br>43,73 % |

(suite)

| i) Phase interne | |
|---|---|
| iii) Phase interne | |
| povidone K 30<br>carboxyméthylcellulose sodique réticulée | 2,53 %<br>5 % |
| Granulation | |
| laurylsulfate de sodium<br>eau purifiée | 0,1 %<br>Q.S. |
| Phase externe | |
| stéarate de magnésium | 1 % |

Les caractéristiques et avantages des compositions selon l'invention apparaitront à la lumière de la description ci-après, à partir des compositions données à titre d'exemple.

ESSAIS

1. Etude de la solubilité des composés de formule (I).

Les solubilités des composés de formule (I) sont mesurées dans différents milieux aqueux. La solubilité instantanée est évaluée, à température ambiante, par dosage. Les résultats exprimés en µg par ml, sont rassemblés dans le tableau 1 ci-après ;

TABLEAU 1

| Composé de formule (I) | Milieu de mise en solution | | | |
|---|---|---|---|---|
| | Eau | Eau + 10 % éthanol | Tampon acétique pH 7.5 | Tampon phosphate pH 7.5 |
| Composé A (base) | 1 | 1.2 | 1,9 | 1.6 |
| Chlorhydrate (solvaté) | 37 | 10 | 54 | 0,5 |
| Méthanesulfonate (solvaté) | 39 | 48 | 54 | 0.9 |
| Hydrogénosulfate | 13 | 38 | 30 | 0.9 |
| Paratoluènesulfonate | 3,9 | 7,3 | 2,4 | 0,2 |
| Phosphate | 1,3 | 7,5 | 0,9 | 0.7 |
| Composé A solvaté | 0,7 | 0,9 | 1,2 | 0,9 |

On a également mesuré la solubilité du composé A dans différents solvants (tableau 2) et après dilution dans l'eau des solutions formées (tableau 3).

TABLEAU 2

| Solvant | Solubilité du composé A |
|---|---|
| Ethanol | 35 mg/ml |
| Polyéthylèneglycol 400 | 50 mg/ml |
| Polyéthylèneglycol 1500 à 60°C | 80 mg/g |

TABLEAU 3

| Solvant | Solubilité du composé A | Dilution dans l'eau | Quantité de composé A dissous | |
|---|---|---|---|---|
| | | | théorique | mesuré |
| Ethanol | 35 mg/ml | 10% | 3,5 mg/ml | $1,2.10^{-3}$ mg/ml |
| Polyéthylèneglycol 400 | 50 mg/ml | 30 % | 15 mg/ml | $3.10^{-3}$ mg/ml |
| Polyéthylèneglycol 1500 à 60°C | 80 mg/g | non diluable | | |

2. Etude de la mouillabilité. La mouillabilité du composé A a été étudiée dans différentes formulations en utilisant la méthode de H. Mohamad et al., Labo Pharma. Problèmes techniques. 1984, 32 (346), 284-289.

2.1. Influence du procédé de granulation.

On a comparé une formulation (formulation 1) obtenue par simple mélange à une formulation (formulation 2) obtenue par granulation humide.

| Formulation 1 | |
|---|---|
| composé A | 30 mg |
| amidon de maïs modifié | 48 mg |
| lactose monohydrate cristaux extra fins | 70,1 mg |
| silice colloïdale anhydre | 0,4 mg |
| stéarate de magnésium | 1,5 mg |
| Gélule | 150 mg |

| Formulation 2 | |
|---|---|
| composé A | 30 mg |
| amidon de maïs modifié | 51 mg |
| lactose monohydrate 200 mesh | 83 mg |
| povidone K 30 | 4,3 mg |
| stéarate de magnésium | 1,7 mg |
| Gélule | 170 mg |

La mouillabilité mesurée selon la méthode de H. Mohamad est 22 $mg^2/s$ pour la formulation 1 et 110 $mg^2/s$ pour la formulation 2.

Ainsi le procédé par granulation humide améliore la mouillabilité d'un facteur 5.

2.2. Influence de la teneur en principe actif.

A titre de comparaison, on a préparé des formulations par granulation humide dans lesquelles la teneur en principe actif est respectivement 10 mg (formulation 3) et 1 mg (formulation 4).

TABLEAU 4

| | Formulation 3 | Formulation 4 |
|---|---|---|
| composé A | 10 mg | 1 mg |
| amidon de maïs | 51 mg | 51 mg |
| lactose monohydrate 200 mesh | 103 mg | 112 mg |
| povidone K 30 | 4,3 mg | 4,3 mg |
| stéarate de magnésium | 1,7 mg | 1,7 mg |
| Gélule | 170 mg | 170 mg |

Pour la formulation 3 la mouillabilité est 500 $mg^2/s$.

Pour la formulation 4 la mouillabilité est 1000 $mg^2/s$.

Ainsi, la mouillabilité est inversement proportionnelle à la quantité de principe actif contenu dans la formule. Ceci illustre le caractère hydrophobe du composé A.

2.3. Influence des excipients

Plusieurs formulations ont été préparées par granulation humide et comparées à une formulation de référence également obtenue par granulation humide.

TABLEAU 5

| | Formulations | | | | |
|---|---|---|---|---|---|
| | | 4 | 5 | 6 | 7 |
| composé A | 30 | 30 | 30 | 30 | 30 |
| amidon de maïs | 51 | 51 | 51 | 51 | 51 |
| lactose monohydrate 200 mesh | 83 | 83 | 83 | 83 | 83 |
| povidone K 30 | 4,3 | 4,3 | 4,3 | 4,3 | 4,3 |
| laurylsulfate de sodium | | 0,17 | 0,85 | | |
| polyéthylèneglycol 6000 | | | | 1,7 | 8,5 |
| stéarate de magnésium | | 1,7 | 1,7 | 1,7 | 1,7 |
| mouillabilité $mg^2/s$ | 600 ±150 | 1200 ±300 | 2300 ±300 | 1100 ±200 | 1100 ±200 |

Seul le laurylsulfate de sodium à 0,5 % améliore la mouillabilité de façon significative.

Les mesures de mouillabilité ne sont pas adaptées pour étudier l'effet d'un agent désintégrant comme la carboxyméftylcellulose sodique réticulée.

3. Etude de la dissolution en milieu gastrique.

On a étudié la cinétique de dissolution de différentes formulations dans un milieu gastrique : à 37°C, dans un tampon phosphate citrate à pH 3, pendant 30 minutes.

Lorsque 40 mg de composé A sont placés seuls dans un litre du milieu de dissolution, on observe qu'aucune quantité n'est dissoute.

Afin de permettre la dissolution des formulations testées, on a ajouté au milieu 0,2 % de laurylsulfate de sodium, à titre de tensioactif.

TABLEAU 6

| Formulations | A | B | C | D |
|---|---|---|---|---|
| | mg | mg | mg | mg |
| **Phase interne** | | | | |
| composé A | 30,0 | 30,0 | 30,0 | 30,0 |
| amidon de maïs | 51,0 | 51,0 | 51,0 | 51,0 |
| lactose monohydrate 200 mesh | 83,0 | 83,0 | 83,0 | 83,0 |
| povidone K 30 | 4,3 | 4,3 | 4,3 | 4,3 |
| laurylsulfate de sodium | 0,17 | 0,85 | | |
| polyéthylèneglycol 6000 | | | 1,7 | 8,5 |
| carboxyméthylcellulose sodique réticulée | | | | |
| eau purifiée pour mouillage | QS | QS | QS | QS |
| **Phase externe** | | | | |
| magnésium stéarate | 1,7 | 1,7 | 1,7 | 1,7 |
| **contenu de la gélule** | **170,17 mg** | **170,85 mg** | **171,7 mg** | **178,5 mg** |

TABLEAU 6 (suite)

| Formulations | E | F | G |
|---|---|---|---|
| | mg | mg | mg |
| **Phase interne** | | | |
| composé A | 30,0 | 30,0 | 30,0 |
| amidon de maïs | 51,0 | 51,0 | 51,0 |
| lactose monohydrate 200 mesh | 83,0 | 83,0 | 83,0 |
| povidone K 30 | 4,3 | 4,3 | 4,3 |
| laurylsulfate de sodium | | 0,17 | |
| polyéthylèneglycol 6000 | | | |
| carboxyméthylcellulose sodique réticulée | 8,5 | 8,5 | 4,25 |
| eau purifiée pour mouillage | QS | QS | QS |
| **Phase externe** | | | |
| magnésium stéarate | 1,7 | 1,7 | 1,7 |
| **contenu de la gélule** | **178,5 mg** | **178,67 mg** | **174,25 mg** |

Pour chaque formulation, on a effectué 6 essais et on a mesuré toutes les 5 minutes la quantité de composé A dissoute dans le milieu.

Le tableau 7 indique la valeur moyenne du pourcentage de composé A dissous et l'écart moyen par rapport à cette valeur pour les différentes formulations décrites dans le tableau 6.

TABLEAU 7

| | Composé A dissous en % (écart moyen) | | | |
|---|---|---|---|---|
| Temps minutes | A | B | C | D |
| 5 | 63,4 (12,8) | 63,5 (24,6) | 56,3 (20,1) | 61,9 (17,0) |
| 10 | 87,9 (13,6) | 87,6 (13.5) | 76,2 (15,6) | 74,7 (15,6) |
| 15 | 97,6 (7,9) | 94,6 (9,7) | 86,9 (13,5) | 81.6 (16,0) |
| 20 | 100,7 (5,4) | 96,6 (7,7) | 93,8 (11.3) | 88,1 (16,0) |
| 25 | 102,1 (4,2) | 98,6 (5,6) | 97,8 (7,7) | 92,1 (15,2) |
| 30 | 103,0 (3,2) | 99,8 (3.7) | 100.2 (5,1) | 94.8 (14.2) |

TABLEAU 7 (suite)

| | Composé A dissous en % (écart moyen) | | |
|---|---|---|---|
| Temps minutes | E | F | G |
| 5 | 62,1 (8,5) | 64,9 (6,4) | 69,8 (7,2) |
| 10 | 85,9 (10,0) | 96,7 (4,5) | 95,4 (8,8) |
| 15 | 97,6 (5,9) | 99.8 (2,3) | 100.4 (5,2) |
| 20 | 100,9 (2,8) | 100,7 (2,1) | 102,8 (2,8) |
| 25 | 101.6 (2,7) | 101,3 (1,9) | 103,8 (1,7) |
| 30 | 102,1 (2,8) | 101,7 (1,5) | 104,2 (1,6) |

Pour les formulations C et D qui contiennent respectivement 1 % et 5 % de polyéthylèneglycol 6000, on constate que l'on n'atteint la dissolution maximum qu'après 30 minutes.

Pour les formulations A et B qui contiennent respectivement 0,1 % et 0,5 % de laurylsulfate de sodium, on observe que la valeur maximale est atteinte respectivement après 20 minutes et 30 minutes.

De plus, les résultats mesurés sont dispersés pour chacune des formulations A, B, C ou D.

Les résultats observés avec les formulations E, F, G montrent l'intérêt de la présence de carboxyméthylcellulose sodique réticulée pour favoriser la dissolution.

Avec les formulations E et G contenant respectivement 5 % et 2,5 % de carboxyméthylcellulose sodique réticulée, on observe que 100 % du composé A est dissous respectivement après 20 minutes ou 15 minutes et que les résultats sont relativement dispersés dans les 15 premières minutes.

La formulation F qui contient à la fois 0,1 % de laurylsulfate de sodium et 5 % de carboxyméthylcellulose sodique réticulée donne les meilleurs résultats. En effet, après 15 minutes la totalité du composé A est dissous, de plus l'écart entre les résultats des différents essais est très faible (écart de 2,3 à 1,5 entre 15 et 30 minutes).

5. Evaluation du passage transépithélial intestinal du composé A.

Sur des filtres microporeux en polycarbonate recouverts de collagène, on ensemence des cellules Caco-2. La monocouche cellulaire formée sur le filtre permet alors de séparer un compartiment apical (mimant la lumière intestinale) d'un compartiment basal (mimant la circulation sanguine).

On place du côté apical la composition contenant le composé à étudier et on évalue le passage de ce composé,

dispersé ou solubilisé dans le milieu de Hank, à travers cette barrière cellulaire en mesurant sa cinétique d'apparition du côté basal. Ce milieu aqueux, de pH = 6,5, a la composition suivante : NaCl = 8,0 g/l ; KCl = 0,4 g/l ; $CaCl_2$ = 0,19 g/l; $MgCl_2$ = 0,1 g/l ; $MgSO_4$ = 0,1 g/l ; $Na_2HPO_4$ = 0,09 g/l ; $KH_2PO_4$ = 0,06 g/l ; $NaHCO_3$ = 0,35 g/l ; glucose = 1 g/l ; rouge phénol = 0,01 g/l.

On détermine ensuite le coefficient de perméabilité P, en cm/s, qui caractérise la vitesse de passage de la molécule à travers la membrane à savoir :

$$P = (da/dt).(1/A.Co)$$

dans lequel :

da/dt = variation de la quantité de composé testé traversant la monocouche cellulaire en fonction du temps (mole/s)
A = surface de la monocouche ($cm^2$)
Co = concentration initiale du composé testé (mole/l)

3.1. Coefficient de perméabilité du composé A introduit dans le milieu de Hank en solution dans le DMSO.
P=$96.10^{-7}$ cm/s.

La perméabilité du composé A ainsi mesurée en solution (dans le DMSO) indique une caractéristique intrinsèque de ce composé. Ce résultat indique la très bonne aptitude du composé A au passage transépithélial lorsqu'il est en solution.

3.2. Vitesse relative du passage transépithélial intestinal du composé A.

On a mesuré la vitesse de passage du composé A dans la formulation X et on l'a comparée à celle du composé A en suspension.

| Formulation X | |
|---|---|
| composé A | 30 mg |
| amidon de maïs modifié | 51 mg |
| lactose monohydrate 200 mesh | 83 mg |
| povidone K 30 | 4,3 mg |
| laurylsulfate de sodium | 0,17 mg |
| carboxyméthylcellulose sodique réticulée | 8,5 mg |
| stéarate de magnésium | 1,7 mg |
| Gélule | 178,67 mg |

| Formulation du composé A | vitesse de passage relative |
|---|---|
| Composé A en suspension dans le milieu de Hank | 1 |
| Composé A formulé dans X | 7 |

EXEMPLE 1 : Gélule à 1 mg

Une gélule préparée par granulation humide ayant la composition suivante :

| Phase interne | |
|---|---|
| composé A micronisé | 1 mg |
| amidon de maïs | 51 mg |
| lactose monohydrate 200 mesh | 103,33 mg |
| povidone K 30 | 4,3 mg |
| carboxyméthylcellulose sodique réticulée | 8,5 mg |

(suite)

| Phase interne | |
|---|---|
| Granulation | |
| laurylsulfate de sodium<br>eau purifiée | 0,17 mg<br>Q.S. |
| Phase externe | |
| stéarate de magnésium<br>Pour une gélule blanc opaque de taille 3 terminée à | 1,7 mg<br>170 mg |

EXEMPLE 2 : Gélule à 10 mg

Une gélule préparée par granulation humide ayant la composition suivante :

| Phase interne | |
|---|---|
| composé A micronisé<br>amidon de maïs<br>lactose monohydrate 200 mesh<br>povidone K 30<br>carboxyméthylcellulose sodique réticulée | 10 mg<br>51 mg<br>94,33 mg<br>4,3 mg<br>8,5 mg |
| Granulation | |
| laurylsulfate de sodium<br>eau purifiée<br>Phase externe | 0,17 mg<br>Q.S. |
| stéarate de magnésium | 1,7 mg |
| Pour une gélule blanc opaque de taille 3 terminée à | 170 mg |

EXEMPLE 3 : Gélule à 30 mg

Une gélule préparée par granulation humide ayant la composition suivante :

| Phase interne | |
|---|---|
| composé A micronisé<br>amidon de maïs<br>lactose monohydrate 200 mesh<br>povidone K 30<br>carboxyméthylcellulose sodique réticulée | 30 mg<br>51 mg<br>74,33 mg<br>4,3 mg<br>8,5 mg |
| Granulation | |
| laurylsulfate de sodium<br>eau purifiée | 0.17 mg<br>Q.S. |
| Phase externe | |
| stéarate de magnésium<br>Pour une gélule blanc opaque de taille 3 terminée à | 1,7 mg<br>170 mg |

EXEMPLE 4 : Gélule à 30 mg

Une gélule préparée par granulation humide ayant la composition suivante

| Phase interne | |
|---|---|
| composé A micronisé<br>amidon de maïs<br>lactose monohydrate 200 mesh<br>povidone K 30<br>carboxyméthylcellulose sodique réticulée | 30 mg<br>51 mg<br>73,65 mg<br>4,3 mg<br>8,5 mg |
| Granulation | |
| laurylsulfate de sodium<br>eau purifiée | 0,85 mg<br>Q.S. |
| Phase externe | |
| stéarate de magnésium<br>Pour une gélule blanc opaque de taille 3 terminée à | 1,7 mg<br>170 mg |

EXEMPLE 5 : Comprimé à 1 mg

| Phase interne | |
|---|---|
| composé A micronisé<br>amidon de maïs<br>lactose monohydrate 200 mesh<br>hydroxypropylméthylcellulose 6 cP<br>carboxyméthylcellulose sodique réticulée | 1 mg<br>50 mg<br>130 mg<br>6 mg<br>10 mg |
| Granulation | |
| laurylsulfate de sodium<br>eau purifiée | 1 mg<br>Q.S. |
| Phase externe | |
| stéarate de magnésium | 2 mg |
| Pour un comprimé terminé à | 200 mg |

EXEMPLE 6 : Comprimé à 10 mg

| Phase interne | |
|---|---|
| composé A micronisé<br>amidon de maïs<br>lactose monohydrate 200 mesh<br>hydroxypropylméthylcellulose 6 cP<br>carboxyméthylamidon sodique<br>laurylsulfate de sodium | 10 mg<br>50 mg<br>211,5 mg<br>9 mg<br>15 mg<br>1,5 mg |
| Granulation | |
| eau purifiée | Q.S. |
| Phase externe | |
| stéarate de magnésium<br>Pour un comprimé terminé à | 3 mg<br>300 mg |

EXEMPLE 7: Comprimé à 30 mg

| Phase interne | |
|---|---|
| composé A micronisé<br>amidon de maïs | 30 mg<br>80 mg |

| | |
|---|---|
| lactose monohydrate 200 mesh<br>povidone K 30<br>carboxyméthylcellulose sodique réticulée<br>laurylsulfate de sodium | 252mg<br>12 mg<br>20 mg<br>2 mg |
| Granulation | |
| eau purifiée | Q.S. |
| Phase externe | |
| stéarate de magnésium<br>Pour un comprimé terminé à | 4 mg<br>400 mg |

**Revendications**

1. Composition pharmaceutique pour l'administration orale du N-pipèridino-5-(4-chlorophényl)-1-(2,4-dichlorophényl)-4-méthylpyrazole-3-carboxamide de formule :

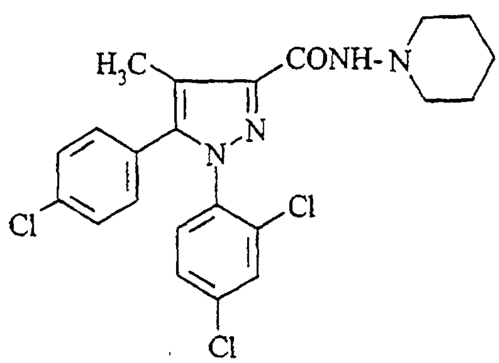

,

d'un de ses sels pharmaceutiquement acceptables ou d'un de leurs solvates (ci-après principe actif), comprenant:

- 0,5 % à 20 % en poids de principe actif sous forme micronisée,
- 0,05 % à 0,5 % en poids d'un alkylsulfate de sodium,
- 2,5 % à 10 % en poids d'agent de désagrégation,

et des excipients pharmaceutiques, ladite composition étant formulée par granulation humide.

2. Composition pharmaceutique selon la revendication 1, comprenant:

- 0,5 % à 20 % en poids de N-pipéridino-5-(4-chlorophényl)-1-(2,4-dichloro phényl)-4-méthylpyrazole-3-carboxamide sous forme micronisée,
- 0,05 % à 0,5 % en poids de laurylsulfate de sodium,
- 2,5 % à 10 % en poids de carboxyméthylcellulose sodique réticulée.

3. Composition pharmaceutique selon la revendication 1, contenant:

- 0,5 % à 20 % en poids de N-pipéridino-5-(4-chlorophényl)-1-(2,4-dichloro phényl)-4-rnéthylpyrazole-3-carboxamide sous forme micronisée,
- 0,1 % en poids de laurylsulfate de sodium,
- 5 % en poids de carboxyméthylcellulose sodique réticulée,
- 1 % à 10 % en poids de liant,
- 0,2 % à 5 % en poids de lubrifiant,

et un diluant en quantité suffisante pour 100 %.

**4.** Composition pharmaceutique selon l'une quelconque des revendications 1 à 3, dans laquelle l'alkylsulfate de sodium est ajouté à l'eau purifiée pour la granulation humide.

**5.** Composition pharmaceutique selon l'une quelconque des revendications 1 à 4, sous forme de gélules, de comprimés, de sachets ou de poudres.

**6.** Composition pharmaceutique selon la revendication 1 sous forme de gélule, ayant la formulation suivante, exprimée en pourcentage pondéral :

| | |
|---|---|
| Phase interne | |
| N-pipéridino-5-(4-chlorophényl)-1-(2,4-dichlorophényl)-4-méthylpyrazole-3-carboxamide sous forme micronisée | 0,59 % |
| amidon de maïs | 30 % |
| lactose monohydrate 200 mesh | 60,78 % |
| povidone K 30 | 2,53 % |
| carboxyméthylcellulose sodique réticulée | 5 % |
| Granulation | |
| laurylsulfate de sodium | 0,1 % |
| eau | Q.S. |
| Phase externe | |
| stéarate de magnésium | 1 % |

**7.** Composition pharmaceutique selon la revendication 1 sous forme de gélule, ayant la formulation suivante, exprimée en pourcentage pondéral :

| | |
|---|---|
| Phase interne | |
| N-pipéridino-5-(4-chlorophényl)-1-(2,4-dichlorophényl)-4-méthylpyrazole-3-carboxamide sous forme micronisée | 5,88 % |
| amidon de maïs | 30 % |
| lactose monohydrate 200 mesh | 55,49 % |
| povidone K 30 | 2.53 % |
| carboxyméthylcellulose sodique réticulée | 5 % |
| Granulation | |
| laurylsulfate de sodium | 0,1 % |
| eau | Q.S. |
| Phase externe | |
| stéarate de magnésium | 1 % |

**8.** Composition pharmaceutique selon la revendication 1 sous forme de gélule, ayant la formulation suivante, exprimée en pourcentage pondéral :

| Phase interne | |
|---|---|
| N-pipéridino-5-(4-chlorophényl)-1-(2,4-dichlorophényl)-4-méthylpyrazole-3-carboxamide sous forme micronisée<br>amidon de maïs<br>lactose monohydrate 200 mesh<br>povidone K 30<br>carboxyméthylcellulose sodique réticulée | 17,64 %<br>30%<br>43,73 %<br>2,53 %<br>5 % |
| Granulation | |
| laurylsulfate de sodium<br>eau | 0,1 %<br>Q.S. |
| Phase externe | |
| stéarate de magnésium | 1 % |

**9.** Procédé pour la préparation d'une composition pharmaceutique selon l'une quelconque des revendications 1 à 5, caractérisé en ce que :

a) on mélange à température ambiante le principe actif, l'agent de désagrégation et l'alkylsulfate de sodium avec un diluant, un liant et éventuellement un colorant ;
b) on mouille le mélange avec de l'eau purifiée ;
c) on sèche et on calibre la masse humide ainsi obtenue ;
d) aux grains secs calibrés ainsi obtenus on ajoute un lubrifiant, et éventuellement un antiadhérent, un agent d'écoulement, un colorant et/ou un aromatisant.

**10.** Procédé selon la revendication 9, pour la préparation d'une composition pharmaceutique selon l'une des revendications 6 à 8, caractérisé en ce qu'on incorpore l'alkylsulfate de sodium à l'étape b).

## Claims

**1.** A pharmaceutical composition for the oral administration of N-piperidino-5-(4-chlorophenyl)-1-(2,4-dichlorophenyl)-4-methylpyrazole-3-carboxamide of the formula

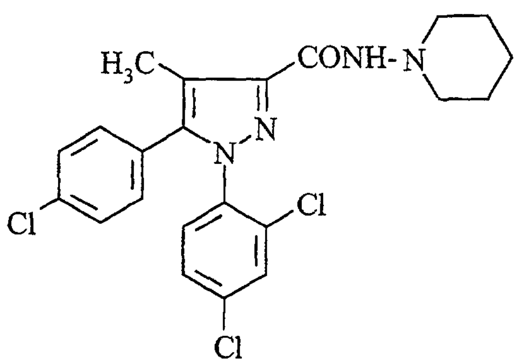

,

of one of its pharmaceutically acceptable salts or of one of their solvates (active principle hereafter), comprising:

- 0.5% to 20% by weight of active principle in micronized form,
- 0.05% to 0.5% by weight of a sodium alkylsulfate,
- 2.5% to 10% by weight of disintegrating agent,

and pharmaceutical excipients, said composition being formulated by wet granulation.

2. A pharmaceutical composition according to claim 1 which comprises:

- 0.5% to 20% by weight of N-piperidino-5-(4-chlorophenyl)-1-(2,4-dichlorophenyl)-4-methylpyrazole-3-carboxamide in micronized form,
- 0.05% to 0.5% by weight of sodium laurylsulfate, and
- 2.5% to 10% by weight of crosslinked sodium carboxymethyl cellulose.

3. A pharmaceutical composition according to claim 1 which contains:

- 0.5% to 20% by weight of N-piperidino-5-(4-chlorophenyl)-1-(2,4-dichlorophenyl)-4-methylpyrazole-3-carboxamide in micronized form,
- 0.1% by weight of sodium laurylsulfate,
- 5% by weight of crosslinked sodium carboxymethyl cellulose,
- 1% to 10% by weight of binder,
- 0.2% to 5% by weight of lubricant,

and a diluent in a sufficient amount for 100%.

4. A pharmaceutical composition according to any one of claims 1 to 3 in which the sodium alkylsulfate is added to the purified water for wet granulation.

5. A pharmaceutical composition according to any one of claims 1 to 4 in the form of gelatin capsules, tablets, sachets or powders.

6. A pharmaceutical composition according to claim 1 in the form of a gelatin capsule and having the following formulation, expressed in percentages by weight:

| | |
|---|---|
| Internal phase | |
| N-piperidino-5-(4-chlorophenyl)-1-(2,4-dichlorophenyl)-4-methylpyrazole-3-carboxamide in micronized form | 0.59% |
| corn starch | 30% |
| 200 mesh lactose monohydrate | 60.78% |
| povidone K 30 | 2.53% |
| crosslinked sodium carboxymethyl cellulose | 5% |
| Granulation | |
| sodium laurylsulfate | 0.1% |
| water | QS |
| External phase | |
| magnesium stearate | 1% |

7. A pharmaceutical composition according to claim 1 in the form of a gelatin capsule and having the following formulation, expressed in percentages by weight:

| | |
|---|---|
| Internal phase | |
| N-piperidino-5-(4-chlorophenyl)-1-(2,4-dichlorophenyl)-4-methylpyrazole-3-carboxamide in micronized form | 5.88% |
| corn starch | 30% |
| 200 mesh lactose monohydrate | 55.49% |
| povidone K 30 | 2.53% |
| crosslinked sodium carboxymethyl cellulose | 5% |
| Granulation | |
| sodium laurylsulfate | 0.1% |

(continued)

| | |
|---|---|
| Internal phase | |
| Granulation | |
| water | QS |
| External phase | |
| magnesium stearate | 1% |

**8.** A pharmaceutical composition according to claim 1 in the form of a gelatin capsule and having the following formulation, expressed in percentages by weight:

| | |
|---|---|
| Internal phase | |
| N-piperidino-5-(4-chlorophenyl)-1-(2,4-dichlorophenyl)-4-methylpyrazole-3-carboxamide in micronized form<br>corn starch<br>200 mesh lactose monohydrate<br>povidone K 30<br>crosslinked sodium carboxymethyl cellulose | 17.64%<br>30%<br>43.73%<br>2.53%<br>5% |
| Granulation | |
| sodium laurylsulfate<br>water | 0.1%<br>QS |
| External phase | |
| magnesium stearate | 1% |

**9.** A process for the preparation of a pharmaceutical composition according to any one of claims 1 to 5, characterised in that:

a) the active principle, the disintegrating agent and the sodium alkylsulfate are mixed at room temperature with a diluent, a binder and, optionally, a colouring agent;
b) the mixture is wetted with purified water;
c) the resulting wet mass is dried and graded; and
d) a lubricant and, optionally, an anti-adhesive, a flowing agent, a colouring agent and/or a flavouring agent are added to the resulting graded dry grains.

**10.** A process according to claim 9 for the preparation of a pharmaceutical composition according to one of claims 6 to 8, characterised in that the sodium alkylsulfate is incorporated in step b).

**Patentansprüche**

**1.** Pharmazeutische Zusammensetzung zur oralen Verabreichung von N-Piperidino-5-(4-chlorphenyl)-1-(2,4-dichlorphenyl)-4-methylpyrazol-3-carboxamid der Formel

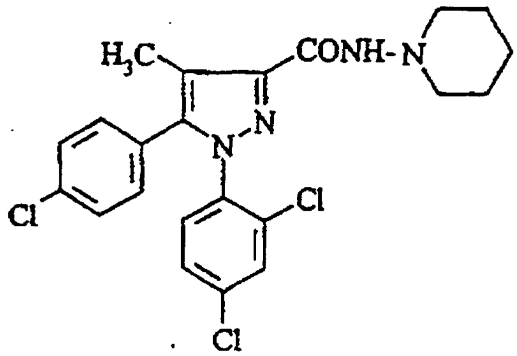

,

eines seiner pharmazeutisch annehmbaren Salze oder eines der Solvate derselben (im folgenden Wirkstoff), umfassend:

- 0,5 bis 20 Gew.-% Wirkstoff in mikronisierter Form,
- 0,05 bis 0,5 Gew.-% eines Natriumalkylsulfats,
- 2,5 bis 10 Gew.% eines zerfallsfördernden Mittels

und pharmazeutische Träger, wobei die Zusammensetzung durch Nassgranulation formuliert wird.

**2.** Pharmazeutische Zusammensetzung nach Anspruch 1, umfassend:

- 0,5 bis 20 Gew.-% N-Piperidino-5-(4-chlorphenyl)-1-(2,4-dichlorphenyl)-4-methylpyrazol-3-carboxamid in mikronisierter Form,
- 0,05 bis 0,5 Gew.-% Natriumlaurylsulfat,
- 2,5 bis 10 Gew.-% vernetzte Natriumcarboxymethylcellulose.

**3.** Pharmazeutische Zusammensetzung nach Anspruch 1, enthaltend

- 0,5 bis 20 Gew.-% N-Piperidino-5-(4-chlorphenyl)-1-(2,4-dichlorphenyl)-4-methylpyrazol-3-carboxamid in mikronisierter Form,
- 0,1 Gew.-% Natriumlaurylsulfat,
- 5 Gew.-% vernetzte Natriumcarboxymethylcellulose,
- 1 bis 10 Gew.-% Bindemittel,
- 0,2 bis 5 Gew.-% Gleitmittel

und ein Verdünnungsmittel in ausreichender Menge auf 100 %.

**4.** Pharmazeutische Zusammensetzung nach einem der Ansprüche 1 bis 3, worin Natriumalkylsulfat für die Nassgranulation zu gereinigtem Wasser gegeben wird.

**5.** Pharmazeutische Zusammensetzung nach einem der Ansprüche 1 bis 4 in Kapsel-, Tabletten-, Säckchen- oder Pulverform.

**6.** Pharmazeutische Zusammensetzung nach Anspruch 1 in Kapselform mit der folgenden Formulierung, ausgedrückt in Gewichtsprozent:

| Innere Phase | |
|---|---|
| N-Piperidino-5-(4-chlorphenyl)-1-(2,4-dichlorphenyl)-4-methylpyrazol-3-carboxamid in mikronisierter Form | 0,59 % |
| Maisstärke | 30 % |
| Lactose-Monohydrat 200 Mesh | 60,78 % |
| Povidon K 30 | 2,53 % |

(fortgesetzt)

| | |
|---|---|
| Innere Phase | |
| vernetzte Natriumcarboxymethylcellulose | 5 % |
| Granulation | |
| Natriumlaurylsulfat<br>Wasser | 0,1 %<br>q.s. |
| Äußere Phase | |
| Magnesiumstearat | 1% |

**7.** Pharmazeutische Zusammensetzung nach Anspruch 1 in Kapselform mit der folgenden Formulierung, ausgedrückt in Gewichtsprozent:

| | |
|---|---|
| Innere Phase | |
| N-Piperidino-5-(4-chlorphenyl)-1-(2,4-dichlorphenyl)-4-methylpyrazol-3-carboxamid in mikronisierter Form<br>Maisstärke<br>Lactose-Monohydrat 200 Mesh<br>Povidon K 30<br>vernetzte Natriumcarboxymethylcellulose | 5,88 %<br><br>30 %<br>55,49 %<br>2,53 %<br>5 % |
| Granulation | |
| Natriumlaurylsulfat<br>Wasser | 0,1 %<br>q.s. |
| Äußere Phase | |
| Magnesiumstearat | 1% |

**8.** Pharmazeutische Zusammensetzung nach Anspruch 1 in Kapselform mit der folgenden Formulierung, ausgedrückt in Gewichtsprozent:

| | |
|---|---|
| Innere Phase | |
| N-Piperidino-5-(4-chlorphenyl)-1-(2,4-dichlorphenyl)-4-methylpyrazol-3-carboxamid in mikronisierter Form<br>Maisstärke<br>Lactose-Monohydrat 200 Mesh<br>Povidon K 30<br>vernetzte Natriumcarboxymethylcellulose | 17,64 %<br><br>30 %<br>43,73 %<br>2,53 %<br>5 % |
| Granulation | |
| Natriumlaurylsulfat<br>Wasser | 0,1 %<br>q.s. |
| Äußere Phase | |
| Magnesiumstearat | 1 % |

**9.** Verfahren zur Herstellung einer pharmazeutischen Zusammensetzung nach einem der Ansprüche 1 bis 5, dadurch gekennzeichnet, dass:

a) der Wirkstoff, das zerfallsfördernde Mittel und Natriumalkylsulfat mit einem Verdünnungsmittel, einem Bindemittel und gegebenenfalls einem Farbstoff bei Umgebungstemperatur gemischt werden;
b) die Mischung mit gereinigtem Wasser angefeuchtet wird;
c) die so erhaltene feuchte Masse getrocknet und kalibriert wird;

d) zu den so erhaltenen trockenen kalibrierten Körnchen ein Gleitmittel und gegebenenfalls ein Antihaftmittel, ein Fließmittel, ein Farbstoff und/oder ein Aromastoff gegeben wird.

**10.** Verfahren nach Anspruch 9 zur Herstellung einer pharmazeutischen Zusammensetzung nach einem der Ansprüche 6 bis 8, dadurch gekennzeichnet, dass Natriumalkylsulfat in Stufe b) beigemengt wird.